(19) Europäisches Patentamt

European Patent Office

Office européen des brevets

(11) **EP 0 650 052 B1**

(12) **EUROPÄISCHE PATENTSCHRIFT**

(45) Veröffentlichungstag und Bekanntmachung des Hinweises auf die Patenterteilung:
**05.04.2000 Patentblatt 2000/14**

(51) Int. Cl.[7]: **G01N 33/497**, G01N 5/04, G01N 25/48, G01N 33/24

(21) Anmeldenummer: **94116809.8**

(22) Anmeldetag: **25.10.1994**

(54) **Verfahren zur Bestimmung aerob biologisch entstehender Wärmeenergie**

Method for determining heat quantities produced by an aerobic biological process

Procédé pour déterminer des quantités de chaleur dues à un processus biologique aérobie

(84) Benannte Vertragsstaaten:
**AT BE CH DE DK IT LI NL SE**

(30) Priorität: **26.10.1993 DE 4336497**

(43) Veröffentlichungstag der Anmeldung:
**26.04.1995 Patentblatt 1995/17**

(73) Patentinhaber:
**HERHOF UMWELTTECHNIK GmbH
D-35606 Solms-Niederbiel (DE)**

(72) Erfinder: **Schnorr, Karl-Ernst
D-35633 Lahnau (DE)**

(74) Vertreter:
**Zinnecker, Armin, Dipl.-Ing. et al
Lorenz-Seidler-Gossel,
Widenmayerstrasse 23
80538 München (DE)**

(56) Entgegenhaltungen:
**EP-A- 0 647 604          DE-A- 3 115 807
DE-A- 3 829 018          DE-A- 4 107 340
DE-A- 4 122 943**

- **PATENT ABSTRACTS OF JAPAN vol. 10, no. 232 (P-486), 12.August 1986 & JP-A-61 066142 (KANEKO AGRICULT MACH CO LTD), 4.April 1986,**
- **BIOTEC, Nr. 4, Juli 1993, DE, Seiten 6-9, XP002021064 M. MEIER-SCHNEIDERS, ET AL.: "BIOKALORIMETRIE"**

**Beschreibung**

Die Erfindung betrifft ein Verfahren zur Bestimmung aerob biologisch entstehender Wärmeenergie.

Aus der DE-OS 41 07 340 ist ein Verfahren zum Kompostieren von organischen Stoffen (Rottegut, Substrat) bekannt, bei dem die organischen Stoffe in einem geschlossenen Behälter unter Luftzuführung aerob biologisch abgebaut werden. Dabei kann zumindest ein Teil der Abluft dem Rottegut erneut zugeführt werden. Ferner ist es möglich, dem Rottegut reinen Sauerstoff zuzuführen.

Aus der DE-OS 36 37 393 ist ein Verfahren bekannt, bei dem organische Stoffe unter Luftzuführung in einem geschlossenen Behälter mikrobiell abgebaut werden. Der geschlossene Behälter besitzt eine Bodenfläche, in der eine Vielzahl von Löchern angeordnet ist, die gleichmäßig über die Bodenfläche verteilt sind, so daß die auf der Bodenfläche zu einem Haufwerk aufgeschütteten organischen Stoffe gleichmäßig von der Zuluft durchströmt werden.

Die organischen Stoffe werden mikrobiell abgebaut. Sie werden dabei mit Sauerstoff (aerob) umgesetzt, wodurch Kohlendioxid $CO_2$ und Wasser $H_2O$ entstehen. Das Kohlendioxid und das Wasser werden mit der Abluft aus dem Rottegut und dem geschlossenen Behälter heraustransportiert. Um das Wasser mit der Abluft aus dem geschlossenen Behälter heraustransportieren zu können, muß das in flüssiger Form vorliegende Wasser verdampft werden. Hierfür muß Energie aufgewendet werden. Diese Energie stammt aus dem Rotteprozeß; sie wird von den Mikroorganismen, die den Rotteprozeß bewirken, erzeugt.

Die prioritätsältere, nicht vorveröffentlichte DE-A-43 34 435 und die prioritätsältere, nicht vorveröffentlichte EP-A-06 47 604, die ein Dokument des Standes der Technik im Sinne von Art. 54 (3) EPÜ ist, betreffen ein Verfahren zum Kompostieren von organischen Stoffen (Rottegut), bei dem das Rottegut in einem geschlossenen Behälter unter Luftzuführung biologisch abgebaut wird, wobei zumindest ein Teil der Abluft dem Rottegut erneut zugeführt wird. Um ein derartiges Verfahren ohne Schwierigkeiten durchführen zu können, wird es derart gesteuert, daß die relative Feuchtigkeit in dem Rottegut zwischen einem unteren Grenzwert und einem oberen Grenzwert gehalten wird. Dabei kann die Abluft gekühlt und der darin enthaltene Wasserdampf zumindest zum Teil auskondensiert werden. Ferner kann der Zuluft reiner Sauerstoff und/oder Frischluft beigemischt werden. In der EP-A-0647604 ist beschrieben, daß die Masse der abgebauten Trockensubstanz genauso groß ist wie die Masse des im Behälter erzeugten Wassers. Ferner wird dort angegeben, daß die Menge der erzeugten Energie aus der Masse des erzeugten Wassers (und damit aus der Masse der abgebauten Trockensubstanz) ermittelt werden kann, wobei diese erzeugte Energie ausschließlich dazu verwendet wird, Wasser im Rottegut zu verdampfen, so daß die aus dem Rottegut mit der Abluft heraustransportierte, als Wasserdampf vorliegende Wassermenge ein Maß für die in dem Rottegut erzeugte Energie liefert. Hierbei ist die aus dem Rottegut mit der Abluft herausgeführte Wassermenge genauso groß wie die aus der Abluft auskondensierte Wassermenge.

In der Praxis besteht ein Bedürfnis nach einem Biokalorimeter, mit dem die aerob biologisch entstehende Wärmemenge bestimmt bzw. festgestellt werden kann.

Die bisher durchgeführten Methoden zur Prozeßbilanzierung bei Biofermentern sind mit Nachteilen behaftet. Zum einen verändern sich die Proben in dem Zeitraum zwischen der Probennahme und dem Analysebeginn hinsichtlich ihres tatsächlichen Wassergehaltes. Die entnommene Probe kann Wasser aufnehmen oder Wasser an die Umgebung abgeben, was zu einem falschen Wert bei der Feststellung der Trockensubstanz führt. Zum anderen vergrößern sich die bei kleinen Probemengen entstehenden Fehler entsprechend ihrer Übertragung auf große Mengen. Wenn beispielsweise in einem biofermenter bzw. einem Behälter bzw. einem geschlossenen Behälter 30.000 Kilogramm Substrat kompostiert werden, und wenn daraus eine Probe von einem Kilogramm entnommen wird, ergibt dies ein Verhältnis von 1:30.000. Hieraus wird deutlich, daß zu einer genauen Bilanzierung des Leistungsvermögens eines Biofermenters bzw. Bioreaktors eine technisch und praktisch nicht realisierbare Genauigkeit der Probennahme erforderlich ist.

Um diesen Nachteil zu umgehen, wurde bisher ein sogenanntes DEWAR-Gefäß (Thermosbehälter) benutzt, in dem die Nachteile einer Momentaufnahme durch eine zeitlich begrenzte Kontrolle der Wärmeentwicklung ersetzt wurden. Der Nachteil dieses Systems besteht jedoch darin, daß von dem eine Wärmemenge bildenden Produkt aus Gas- oder Flüssigkeitsmenge, spezifischer Stoffwärme und Temperatur lediglich die Temperatur sowie ein Produkt aus Temperatur und Zeit benutzt wird. Dies gibt verständlicherweise keine exakte physikalische Größe einer thermischen Zustandsänderung.

In der Veröffentlichung "Biokalorimetrie", veröffentlicht in der Zeitschrift Biotec Nr. 4, Juli 1993, werden Meßtechniken beschrieben, die auf dem Prinzip eines kontrollierten Wärmeflusses beruhen, wobei die adiabatische oder isotherme Zustandsänderung im Reaktionsgefäß (Biofermenter, Bioreaktor, (geschlossener) Behälter) auf die Umgebung übertragen und hier gemessen wird.

Dort ist ein Verfahren beschrieben zur Messung der Wärmeenergie in einem Fermenter. Der Fermenter ist von einem Kühlmantel umgeben. Proportional zur Temperaturdifferenz zwischen Fermenter und Kühlmantel wird dem Fermenter ein konstanter Wärmestrom entzogen. Wird keine Fermentationswärme freigesetzt, so muß dieser Wärmestrom vollständig durch eine elektrische Kompensationsheizung bereitgestellt werden. Jede Wärmeerzeugung im Fermenter führt somit zu einer Erniedrigung der elektrischen Heizleistung. Diese Änderung der Leistung der Kompensationsheizung stellt das Meßsignal dar. Das Verfahren nach der genannten Veröffentlichung ist mit einem verhältnismäßig hohen Aufwand verbunden, und die Genauigkeit der Ergebnisse ist nicht zufriedenstellend.

Aufgabe der Erfindung ist es, ein Verfahren zur Bestimmung aerob biologisch entstehender Wärmeenergie anzugeben, mit dem die biologisch entstehende Wärmemenge zuverlässig und genau bestimmt werden kann.

Erfindungsgemäß wird diese Aufgabe dadurch gelöst, daß die Wärmeenergie aus der Menge bzw. Masse des biologisch gebildeten Wassers bestimmt bzw. ermittelt wird. Die biologisch gebildete Wassermenge kann ihrerseits auf verschiedene Weise ermittelt werden. Beispielsweise kann die biologisch gebildete Wassermenge aus der Feuchtigkeit in der Abluft ermittelt werden. Statt dessen oder zusätzlich kann die biologisch gebildete Wassermenge aus der Gewichtsabnahme des Substrats ermittelt werden. Schließlich kann die Menge des biologisch gebildeten Wassers statt dessen oder zusätzlich aus dem Sauerstoffverbrauch ermittelt werden.

Vorteilhafte Weiterbildungen sind in den weiteren Unteransprüchen beschrieben.

Die Erfindung beruht auf der Erkenntnis, daß Luft, die über Oberflächen von festen Körpern, beispielsweise über das Substrat, streicht, dort nur dann Wasser aufnimmt, wenn sich ihr Wärmeinhalt ändert. Dies folgt aus der Thermodynamik der Luft bzw. der feuchten Luft, wie sie im i-x-Diagramm zum Ausdruck kommt und dargestellt werden kann. Wenn keine aus biochemischer Reaktion entstehende Wärme von den Festkörperoberflächen auf die Luft übertragen wird, kühlt diese Luft aus. Wählt man daher für die Luft die gleiche Temperatur wie für die Festkörperoberflächen, so kühlt die Luft ab, ohne Wärme zu verlieren, und zwar aufgrund einer adiabaten Reaktion bis zur Sättigungslinie. Wird aus der biochemischen Reaktion Wärme freigesetzt, so nimmt die Luft bei gleichbleibender Temperatur Feuchtigkeit und Wärme auf, und zwar bei einer isothermen Reaktion bis zur Sättigungslinie. Die Temperatur der Luft steigt anschließend entlang der Sättigungslinie bis zu der Temperatur, die für Mikroorganismen maximal noch verkraftbar ist, also bis zur maximal für Mikroorganismen verkraftbaren Temperatur.

Die Erfindung beruht auf der Erkenntnis, daß zur Durchführung einer genauen Bilanzierung (Wärmebilanz bzw. Energiebilanz) undefinierbare Verluste von Stoffwechselprodukten ($CO_2$, $H_2O$ und/oder Wärme) vermieden werden müssen, und daß die biologisch entstehende Wärme als Wasserbildungsenthalpie zur Wasserverdunstung führt. Die anschließend kondensierbare Wassermenge führt über die Bestimmung der Kondensationsenergie zur Verdunstungsenergie, die gleich ist der Wasserbildungsenthalpie minus der Energie zum mikrobiellen Zellaufbau (siehe die bereits erwähnte, früher angemeldete, aber nicht vorveröffentlichte DE-A-43 34 435).

Durch die Erfindung wird ein Biokalorimeter geschaffen, mit dem es möglich ist, die biologisch entstehende Wärmemenge bei der Veratmung von kleinsten Substratmengen festzustellen, indem es gelungen ist, die Zustandsänderung der mit den Mikroorganismen in Verbindung kommenden Luft genau zu ermitteln. Hierbei werden in einer bestimmten Zeit Menge, Temperaturänderung und Wassergehaltsänderung der Luft als variable Zustandsgrößen ermittelt und bilanziert.

Die vorliegende Erfindung kann auf aufwendige Meßsysteme verzichten, da sie von der Grundvoraussetzung ausgeht, daß die Wasserbildungsenthalpie eine konstante Größe darstellt und daß der Wasserverlust des Substrates hierzu proportional verläuft. Hierbei wird vorausgesetzt, daß die Zustandsänderung im Reaktionsgefäß (Biofermenter, Bioreaktor, (geschlossener) Behälter) adiabat und/oder isotherm verläuft. Zur Erfassung der Stoffwechselproduktmenge Wärme wird das Substrat im Reaktionsgefäß von Luft umspült oder durchspült. Dabei muß der Feuchtigkeitsgehalt der aus dem Reaktionsgefäß austretenden Luft stets höher sein als der Feuchtigkeitsgehalt der eintretenden Luft. Die Gewichtsänderung des Substrates zeigt die bzw. liefert ein Maß für die aufgrund der biologischen Reaktion verdunstete Wassermenge. Auch insoweit wird auf die deutsche Patentanmeldung DE-A-43 34 435 verwiesen. Auf experimentellem Wege wurde ermittelt, daß das Gewicht der verbrauchten Sauerstoffmenge gleich ist dem Gewicht der freigesetzten $CO_2$-Menge. Daraus kann abgeleitet werden: "Die Masse von beiden Gasen (also von $O_2$ und $CO_2$) ist im Betriebszustand bei der Temperatur $t_x$ gleich".

Im Normzustand ist die Dichte von $CO_2$ gleich 1,38 mal der Dichte von $O_2$. Bei einem idealen Gas und konstantem Druck beträgt die Temperatur durch Zustandsänderung demnach

$$T_x = 1 \times 1{,}38 \times 273{,}16\ °K - 273{,}16\ °K =$$
$$= 104\ °K = 104\ °C.$$

Diese Temperatur könnte im Moment der sogenannten "biochemischen Knallgasreaktion" entstehen, wenn bei dem Stoffwechsel das Substrat mit Sauerstoff und Wasserstoff chemisch reagiert und Wasser und Kohlendioxid freigesetzt werden.

Aus der Strukturformel für den biologischen Abbau der Glukose ist erkennbar, daß durch Zufuhr von 96 kg Sauerstoff bei Normzustand die Menge von 132 kg Kohlendioxid entsteht und bei einem Betriebszustand von 104 °C sowie 760 Torr die der verbrauchten Sauerstoffmenge äquivalente Menge von 96 kg $CO_2$. Dividiert man die bei Normzustand entstehende $CO_2$-Menge von 132 kg durch 1,38, so erhält man tatsächlich die im Betriebszustand bei 104 °C

feststellbare $CO_2$-Menge. Würde die dabei freigesetzte Wärmemenge nicht abgeführt (beispielsweise durch Wasserverdunstung), so würde der Mikroorganismus an Überhitzung absterben (dies kann bei der Kompostierung, insbesondere nach dem Abbau der leicht abbaubaren Substanz, zur Hygienisierung des Substrats genutzt werden). Die aus der Abluft auskondensierte Wassermenge entspricht der Gewichtsänderung der Substratmenge.

Die Wärmemenge kann beispielsweise dadurch aus der Feuchtigkeit bzw. aus der Menge der Feuchtigkeit in der Abluft ermittelt werden, daß die in der Abluft enthaltene Feuchtigkeit auskondensiert wird.

Die Erfindung beruht auf der Erkenntnis, daß die Gewichtsabnahme des Substrates durch Verdunstung proportional ist der mikrobiell verfügbaren Glukosemenge. Die Gewichtsabnahme des Substrates durch Verdunstung ist ferner proportional der biologisch gebildeten Wassermenge. Die Gewichtsabnahme des Substrates durch Verdunstung ist auch proportional der Menge des Kondensatwassers aus der das Substrat verlassenden Abluft.

Nach einer vorteilhaften Weiterbildung der Erfindung wird die Gewichtsabnahme eines bzw. des Substrates, die gleich ist mit der mit der Spülluft ausgetragenen Kondesatmenge in einer bestimmten Zeit, multipliziert mit der Wasserdampfenthalpie (i") der bei Kondensation feuchtigkeitsgesättigten Spülluft. Diese Wasserdampfenthalpie i" beträgt beispielsweise bei 21 °C 606,5 Kcal/kg.

Die Erfindung betrifft ferner ein Verfahren zur Bestimmung der aerob mikrobiologischen Abbauleistung. Dieses Verfahren ist erfindungsgemäß dadurch gekennzeichnet, daß die mikrobiologische Abbauleistung aus der biologisch entstandenen Wärmemenge bzw. Wärmeenergie bestimmt wird. Die biologisch entstandene Wärmemenge kann dabei nach einem der oben angegebenen, erfindungsgemäßen Verfahren bestimmt werden. Dabei wird die Erkenntnis genutzt, daß die nach Ermittlung der biologischen Wärmeenergie feststellbare biologisch entstandene Wassermenge und ihre Zusammensetzung gleich ist der Änderung der Trockensubstanz sowie der Änderung des Glühverlustes.

Bei den erfindungsgemäßen Verfahren kann ein in aerober Zersetzung befindliches Substrat mit Luft umspült und/oder durchspült werden, wobei die Luftzustandsänderung zwischen Eintritt und Austritt gemessen werden kann. Vorzugsweise wird die Abluft so gekühlt, daß ihr Wassergehalt bei Austritt in die Atomsphäre gleich ist dem Wassergehalt vor Eintritt in das Substrat.

Die erfindungsgemäßen Verfahren können in einem Behälter, insbesondere in einem geschlossenen Behälter, durchgeführt werden. Dabei kann es sich um den Behälter handeln, in dem die Kompostierung durchgeführt wird, also beispielsweise um einen Behälter der aus der DE-OS 36 37 393 bekannten Art. Es kann sich aber auch um einen Probebehälter handeln, in den eine Probe aus dem Kompostierungsbehälter (also der "Rottebox") eingegeben wird.

Wenn die biologisch gebildete Wassermenge aus dem Sauerstoffverbrauch des Substrats ermittelt wird, kann dies dadurch geschehen, daß der Sauerstoffgehalt (der relative Sauerstoffgehalt in %) der Zuluft und der Abluft gemessen werden und daß die zugeführte bzw. abgeführte Sauerstoffmenge bzw. Luftmenge pro Zeiteinheit in der Zuluft und der Abluft gemessen werden.

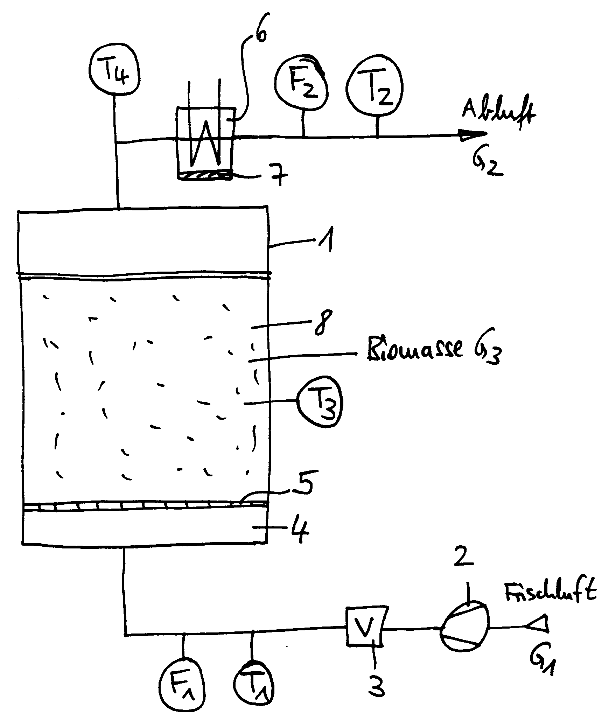

Ein Ausführungsbeispiel der Erfindung wird nachstehend anhand der Zeichnung im einzelnen beschrieben. In der Zeichnung zeigt die

einzige Fig.    ein Schema einer Vorrichtung, mit der die erfindungsgemäßen Verfahren durchgeführt werden können.

Die in der einzigen Figur dargestellte Vorrichtung besteht aus einem geschlossenen Behälter 1, in dem sich organische Stoffe 8 (Rottegut, Biomasse, Substrat) befinden, die auf einem Lochboden 5 aufliegen. Von einem Gebläse 2 wird Frischluft angesaugt. Sie strömt durch einen Durchflußmesser 3, hinter dem die Temperatur T1 und die relative Feuchte F1 der Luft gemessen werden, in den Raum 4 des geschlossenen Behälters 1 unter dem Lochboden 5. Von dort strömt die Luft in dem Behälter 1 nach oben durch die Biomasse hindurch. Die Luft verläßt den geschlossenen Behälter 1 an seinem oberen Ende als Abluft. Dort wird die Temperatur T4 gemessen. Die Luft durchströmt dann einen Wärmetauscher 6 bzw. eine Kühlfalle, in dem sich das Kondensat 7 sammelt. Danach werden die Temperatur 2 und die relative Feuchte F2 der auskondensierten Abluft gemessen. In der Vorrichtung bezeichnet "1" den Zustand der Frischluft, "2" den Zustand der Abluft, "3" den Zustand in der Biomasse und "4" den Zustand der Abluft nach dem Verlassen des Behälters 1 und vor dem Eintritt in den Wärmetauscher 6.

Die aus einem geschlossenen Behälter 1 und einem luftdurchlässigen Boden 5 bestehende Vorrichtung besitzt ferner eine in der Zeichnung nicht dargestellt Wiegevorrichtung, durch die das Gewicht der Biomasse und/oder die Gewichtsabnahme der Biomasse gemessen werden können. Der geschlossene Behälter wird von atmosphärischer Luft durchspült, er kann aber auch von einem anderen Gas durchspült werden. Die Luftmenge wird derart bemessen, daß die Temperatur auf der Abluftseite entweder genauso groß ist oder größer ist als auf der Frischluftseite. Es gilt also

$$T4 \geq T1.$$

Die Abluft tritt anschließend in den Wärmetauscher 6, der auch als Kühlfalle bezeichnet werden kann, ein. Hier wird die Abluft entfeuchtet, und zwar in einem solchen Ausmaß, daß sie nach der Entfeuchtung denselben Wassergehalt wie die Zuluft aufweist. Das auf der Luftaustrittseite (Abluftseite) aufgefangene Kondensat dient auf diese Weise der Berechnung der biologischen Aktivität gemäß folgender Gleichungen:

$$i = i'' + i'$$

mit

i = Bioenthalpie
i'' = Enthalpie des Dampfes
i' = Enthalpie des Kondensats

Ferner gilt

$$H = Q$$

mit

H = Biowärme
Q = Verdampfungswärme

Schließlich gelten noch folgende Zusammenhänge:

$$i'' = r + i'$$

mit

i'' = Enthalpie des Dampfes
r = Verdampfungswärme
i' = Enthalpie des Kondensats

und

$$Q = H = r \times m_K + m_K \times c \times t$$

mit

Q = Verdampfungswärme
H = Biowärme
r = Verdampfungswärme = 585,5 Kcal/kg bei 21 °C
$m_K$ = Masse des Kondensats in Kilogramm
c = spezifische Wärme in Kcal/kg grd
t = Temperatur in °C

Die Enthalpie des Dampfes i'' beträgt 606,5 Kcal/kg bei 21 °C.

Die Bioenthalpie resultiert aus der biochemischen Reaktion der Wasserbildung. Unter der Voraussetzung, daß bei der biologischen Umsetzung von Glukose 57 Kcal pro Mol Wasser entstehen und 1 Mol Wasser 0,018 kg wiegt, entstehen je kg Wasser 3.166,66 Kcal Bioenthalpie ($i_{total}$). Diese Energiemenge steht nicht als freie Wärmemenge zur Verfügung, sondern nur die Teilmenge $i_{Frei}$ = 2905,55 Kcal, da die Differenz für den Zellaufbau der Mikroorganismen verbraucht wird.

Die biologisch gebildete Wärmemenge entspricht somit der biologisch gebildeten Wassermenge, und diese kennzeichnet die Änderung in der Trockensubstanz eines Substrates bzw. die Änderung des Glühverlustes durch biologischen Abbau in einer bestimmten Zeit. Sie kennzeichnet die Leistung des Fermentationssystems und den Rottegrad. Dementsprechend betrifft die Erfindung auch ein Verfahren zur Bestimmung des Rottegrades.

Die aerobe Abbauleistung $P_A$ in kg/h kann (über den Brennwert) wie folgt analytisch bestimmt werden:

$$P_A = \frac{TS_{anf} - TS_{ende}}{h} = \frac{GV_{anf} - GV_{ende}}{h} \ [kg/h]$$

mit

$TS_{anf}$ = Masse (Menge) der Trockensubstanz am Anfang in kg

$TS_{ende}$ = Masse der Trockensubstanz am Ende in kg

h = Zeit in Stunden

$GV_{anf}$ = Glühverlust am Anfang in kg

$GV_{ende}$ = Glühverlust am Ende in kg

Meßtechnisch kann die aerobe Abbauleistung $P_K$ in kg/h (über die Kondensation des Wasserdampfes in der Abluft) wie folgt bestimmt werden:

$$P_K = \frac{H}{i_{Frei} x h} = \frac{(m_k \ x \ r)+(m_k \ x \ c \ x \ t)}{2905,55 \ x \ h} \ [kg/h]$$

mit

H = Biowärme in Kcal (wobei H = Q gilt)

$i_{Frei}$ = freie Enthalpie des biologisch gebildeten Wassers, bezogen auf die Masse in Kcal/kg

h = Zeit in Stunden

$m_K$ = Masse des Kondensats in kg

r = Verdampfungswärme in Kcal

c = Spezifische Wärme in Kcal/kg grd

t = Temperatur in °C

Schließlich kann die aerobe Abbauleistung $P_G$ in kg/h auch noch wie folgt meßtechnisch bestimmt werden (über die Gewichtsdifferenz der Substratmasse):

$$P_G = \frac{(m_{anfang}-m_{ende}) x 606,5}{2905,55 \ x \ h} \ [kg/h]$$

mit

$m_{anfang}$ = Masse des Substrats am Anfang in kg

$m_{ende}$ = Masse des Substrats am Ende in kg

h = Zeit in Stunden

Das Verfahren kann derart ausgeführt werden, daß die Temperatur T2 21 °C beträgt. Die Temperatur T3 sollte derart eingestellt werden, daß gilt

$$T3 = (T1 + T4)/2.$$

Ferner gilt:

$$\Sigma \ \Delta(G_4 - G_1) = G_{3Anf} - G_{3Ende}$$

mit

$G_4$ = Masse der Abluft an der Stelle "4" in kg

$G_1$ = Masse der Frischluft an der Stelle "1" in kg

$\Delta(G_4 - G_1)$= Änderung der Masse der Luft von der Stelle "1" zu der Stelle "4" in kg

$G_3$Anf = Biomasse $G_3$ am Anfang in kg

$G_3$Ende = Biomasse $G_3$ am Ende in kg

Ferner gilt:

$$\Sigma\ \Delta(G_4\text{-}G_1) \times 606,5\ \frac{kcal}{kg} : 2905,6\ \frac{kcal}{kg} = \Delta TS = \Delta GV$$

mit

$\Delta TS$ = Änderung der Trockensubstanz

$\Delta GV$ = Glühverlust

## Patentansprüche

1. Verfahren zur Bestimmung aerob biologisch entstehender Wärmeenergie bei einem Verfahren, bei dem ein in aerober Zersetzung befindliches Substrat mit Luft umspült und/oder durchspült wird, bei dem die biologisch gebildete Wassermenge aus der Feuchtigkeit in der Abluft ermittelt wird, wobei die in der Abluft enthaltene Feuchtigkeit auskondensiert wird, und bei dem die Wärmeenergie aus der Menge des biologisch gebildeten Wassers nach folgender Formel bestimmt wird:

$$Q = H = r \times m_K + m_K \times c \times t$$

worin bedeuten:

Q = Verdampfungswärme
H = Biowärme
r = Verdampfungswärme
$m_K$ = Masse des Kondensats
c = spezifische Wärme
t = Temperatur

2. Verfahren nach Anspruch 1, dadurch gekennzeichnet, daß die Luftzustandsänderung zwischen Luft-Eintritt in das Substrat und Luft-Austritt aus dem Substrat gemessen wird.

3. Verfahren zur Bestimmung aerob mikrobiologischer Abbauleistung bei einem Verfahren, bei dem ein in aerober Zersetzung befindliches Substrat mit Luft umspült und/oder durchspült wird,
   dadurch gekennzeichnet,
   daß die mikrobiologische Abbauleistung aus der aerob biologisch entstandenen Wärmeenergie bestimmt wird, wobei die biologisch entstandene Wärmeenergie nach einem Verfahren nach einem der Ansprüche 1 oder 2 bestimmt wird.

4. Verfahren nach Anspruch 3, dadurch gekennzeichnet, daß die Abluft so gekühlt wird, daß ihr Wassergehalt bei dem Austritt in die Atmosphäre gleich ist dem Wassergehalt vor dem Eintritt in das Substrat.

**EP 0 650 052 B1**

**Claims**

1. Method for determining heat quantities produced by an aerobic biological process in which a substrate undergoing aerobic decomposition is exposed to a surrounding or penetrating flow of air, in which method the biologically formed quantity of water is determined from the moisture content of the exhaust air, with the moisture contained in the exhaust air being separated out by condensation, and in which method the thermal energy is determined from the quantity of biologically formed water in accordance with the following formula:

$$Q = H = r \times m_K + m_K \times c \times t$$

where

    Q = Heat of evaporation
    H = Biologically generated heat
    r = Heat of evaporation
    $m_K$ = Mass of the condensate
    c = Specific heat
    t = Temperature

2. Method according to Claim 1, characterized in that the change in the state of the air from entry in the substrate to exit from the substrate is determined by measurement.

3. Method for determining the rate of aerobic microbiological decomposition in a process in which a substrate undergoing aerobic decomposition is exposed to a surrounding or penetrating flow of air,
characterized in that
the rate of microbiological decomposition is determined from the thermal energy generated by the aerobic biological process, with the relevant heat quantities being determined in accordance with the method according to Claim 1 or 2.

4. Method according to Claim 3, characterized in that the exhaust air is cooled so that its water content on entry into the atmosphere is identical to its water content prior to entry in the substrate.

**Revendications**

1. Procédé pour déterminer des quantités de chaleur dues à un processus biologique aérobie dans lequel un substrat se trouvant en décomposition aérobie est exposé à de l'air l'enveloppant ou le traversant, dans lequel la quantité d'eau biologiquement formée est déterminée à partir de l'humidité dans l'air d'évacuation, l'humidité contenue dans l'air d'évacuation étant séparée par condensation, et dans lequel la quantité de chaleur est déterminée à partir de la quantité d'eau biologiquement formée selon la formule suivante:

$$Q = H = r \times m_K + m_K \times c \times t$$

dans laquelle:

    Q = chaleur d'évaporation
    H = chaleur biologique
    r = chaleur d'évaporation
    $m_K$ = masse du condensat
    c = chaleur spécifique
    t = température.

2. Procédé selon la revendication 1, caractérisé en ce que le changement de l'état de l'air entre l'entrée de l'air dans le substrat et la sortie de l'air du substrat est mesuré.

3. Procédé pour déterminer la puissance de dégradation microbiologique aérobie dans un procédé dans lequel le substrat se trouvant en décomposition aérobie est exposé à de l'air l'enveloppant ou le traversant,
caractérisé en ce que
la puissance de dégradation microbiologique est déterminée à partir de la quantité de chaleur due à un processus

**8**

biologique aérobie, la quantité de chaleur biologiquement formée étant déterminée selon l'une quelconque des revendications 1 ou 2.

4. Procédé selon la revendication 3, caractérisé en ce que l'air d'évacuation est refroidi de manière à ce que sa teneur en eau à la sortie dans l'atmosphère est égale à la teneur en eau avant l'entrée dans le substrat.